# EUROPEAN PATENT SPECIFICATION

(11) **EP 0 535 799 B1**
(45) Date of publication and mention of the grant of the patent: **27.10.1999**
(21) Application number: 92307754.9
(22) Date of filing: 26.08.1992
(51) Int. Cl.: G01N 33/86, C12Q 1/56, G01N 33/48

(54) **Reagent for coagulating blood**
Reagenz zur Blutgerinnung
Reactif pour coaguler le sang

(30) Priority: 30.08.1991 JP 24674091
(43) Date of publication of application: 07.04.1993
(73) Proprietor: SYSMEX CORPORATION, Chuo-ku Kobe 651-0073 (JP)
(72) Inventor: Yonemura, Masaru, Sumaku, Kobe (JP)
(74) Representative: Price, Paul Anthony King

(56) References cited:
- EP-A- 0 062 310
- EP-A- 0 083 773
- EP-A- 0 123 883
- EP-A- 0 241 613
- DE-A- 3 441 835
- FR-A- 2 251 002

## Description

The invention relates to the use of a high molecular weight substance in a reagent for coagulating preferably, but not exclusively, blood, e.g. when measuring the coagulation activity of a plasma specimen by using optical measurement apparatus or viscosity detection apparatus.

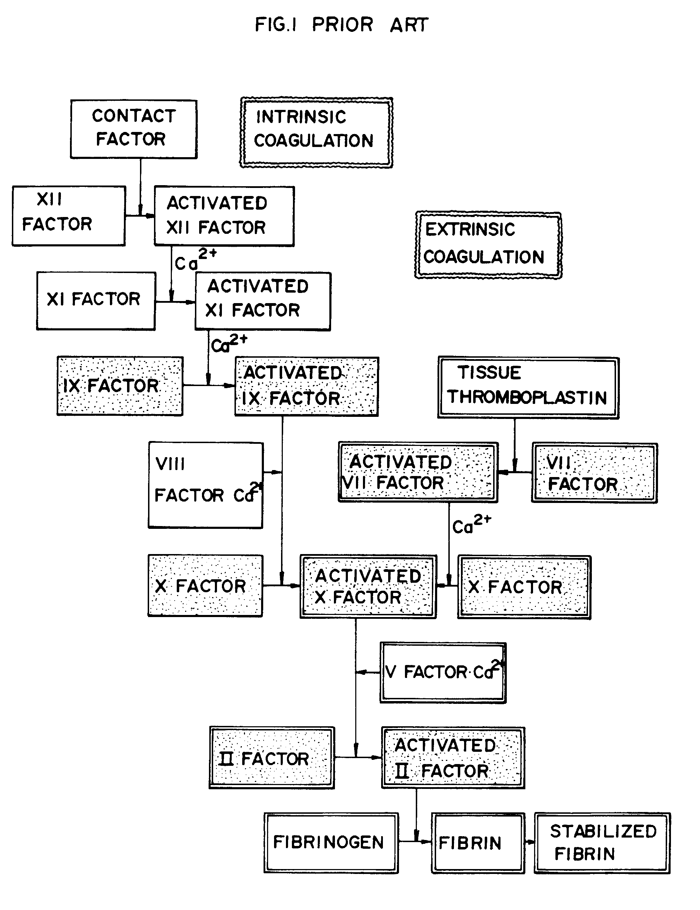

The mechanism of blood coagulation is explained with reference to Fig. 1. Blood can coagulate via two pathways.

One pathway is called extrinsic coagulation. Starting from the release of thromboplastin by epidermic cells or the like, the coagulation factor VII is activated, and the activated coagulation factor VII activates the coagulation factor X. This activates the coagulation factor V and factor II, and finally fibrinogen is transformed into fibrin to induce coagulation.

The other pathway is called intrinsic coagulation. The coagulation factor XII is activated by contact or the like and activates the factor XI. In turn, the activated factor XI activates the factor IX, and the activated factor IX activates the factor X by collaborating with calcium ions and factor VIII. This is then followed by activation of the factor V and factor II, and finally fibrinogen is transformed into fibrin to induce coagulation.

Methods of detecting blood coagulation include the method of detecting the increase in viscosity (viscosity detection method), the method of detecting white turbidity (turbidity detection method), and their combined method. In the viscosity detection method, a bar-shaped or spherical magnetic element is placed in the plasma specimen, and a coagulation reagent is added. The motion of the magnetic element becomes slower due to coagulation, and this slowing down is detected. The viscosity detection method produces variable results depending on the shape of the fibrin lumps which are the final product of blood coagulation (that is, the quantity or viscosity of the fibrin). Furthermore, it is impossible to detect coagulation unless the viscosity increases to above a specific level. Besides, because the measurement principle is based on observing the motion of the magnetic element, it is dependent on the strength of the magnetic field of the element.

The turbidity detection method involves mixing the plasma specimen and coagulation reagent, and it does not require a magnetic element or the like. The method can be the transmitted light detection method or the scattered light detection method. With these methods of detection, if the fibrinogen quantity is small, the change in the quantity of transmitted or scattered light can be detected, and it is hence free from the shortcoming of the viscosity detection method. However, with either method, the detection is more accurate if the change in the quantity of light is large. Thus the coagulation reagent should have the characteristic of producing a large change in the quantity of light.

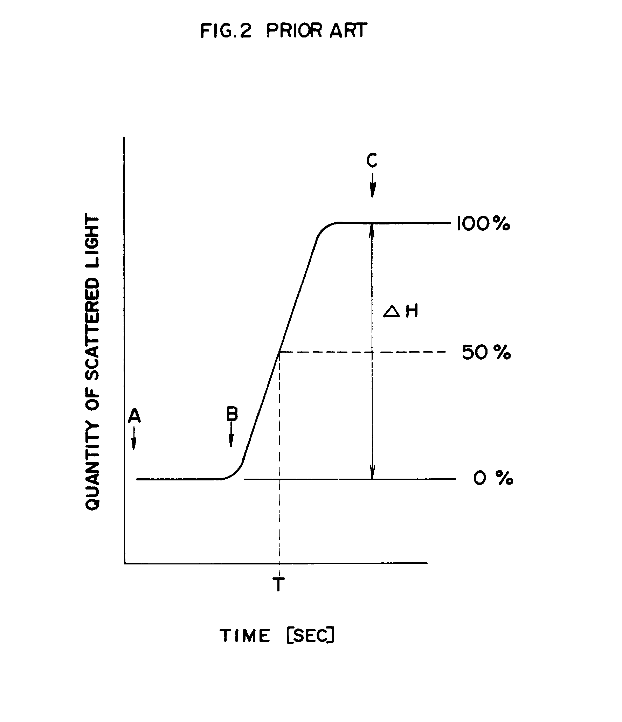

Fig. 2 is a diagram for explaining the change of signal intensity obtained in the scattered light detection method. The result of investigating the plasma coagulation process with an optical detecting device (using the scattered light detection method) is shown. In the diagram, point A is the moment of mixing the plasma and coagulation reagent, and afterwards the coagulation reaction progresses in multiple steps. As stable fibrin is formed, a change appears in the quantity of scattered light (point B in the diagram). As the formation of stable fibrin progresses, the change in the quantity of scattered light increases. When most of the fibrinogen has been consumed, the change in the quantity of scattered light stops increasing, and the reaction terminates (point C). The coagulation time may be (for example as disclosed in Japanese Laid-open Patent Sho. 59-203959) defined as the time T taken to reach 50% of the overall change in the quantity of scattered light, with time B being 0% and time C being 100%. ΔH is the overall change in the quantity of scattered light from start to finish of the coagulation reaction.

To measure the pathway of extrinsic coagulation, the method known as Quick's single-stage method is the most popular at present. This measurement or examination is generally called the PT (prothrombin time) method, and is capable of not only measuring comprehensively the activity of extrinsic coagulation factors, but also measuring individual extrinsic coagulation factors by using coagulation factor deficient plasma. Today, for patient-administered anticoagulants (such as warfarin) for the treatment of heart disease, this measurement method is important as the means of monitoring the effect of the anticoagulants.

Most reagents used in this method are prepared by grinding rabbit brain in the presence of acetone, and then dehydrating, drying and extracting tissue thromboplastin. The tissue thromboplastin differs in reactivity to extrinsic coagulation factors or yield depending on the extraction conditions, and various methods of extraction have been invented so far. For example, Ronald Bach et al. attempted to enhance the yield by solubilizing the tissue thromboplastin by using surface active agent (Triton X-100) (The J. Biological Chemistry, Vol. 256, No. 16, pp. 8324-8331, 1981). As a similar method, in Japanese Patent Publication Sho. 63-56501, it is proposed to use surface active agent of the cholic acid group at the time of tissue thromboplastin extraction. Concerning these methods, M. Hvatum and H. Prydz (Biochimica et Biophysica Acta, Vol. 130, pp 92-101, 1966) and H. Gonmori and Takeda (Thrombos. Haemostas. Vol 36, pp. 90-130, 1976) reported the method of preparing tissue thromboplastin in detail in their papers. Besides, Japanese Patent Publication Sho. 58-43080 discloses an invention for preparing a useful tissue thromboplastin by maintaining an alkaline pH during extraction.

Whichever method of extraction is selected, it is essential that the tissue thromboplastin is contained in the reagent used in the inspection method (prothrombin time method) known as Quick's single-stage method, and at the same time the tissue thromboplastin must be uniform in reactivity to extrinsic coagulation factors and must also be able to transform fibrinogen into fibrin.

For the conventional reagent for coagulating blood (hereinafter called PT reagent), it was the overriding requirement to have an accurate reactivity to extrinsic coagulation factors and the ability to form fibrin. Little attention has been paid to displaying accurate measurement results. It was necessary to allow significant fluctuations of the coagulation results.

The present invention involves the use of a high molecular weight substance of molecular weight between 1,000 and 2,000,000 in a reagent for coagulating blood comprising a substance activating coagulation factor and calcium ions for increasing turbidity changes when said reagent is added to a plasma sample.

Embodiments of the invention produce a large change in the quantity of transmitted or reflected light in order to improve the measurement accuracy. As a result, not only can a specimen of low coagulating activity be measured, but the accuracy of the measurement of normal specimens is also enhanced.

Embodiments produce enhanced optical and viscosity changes. The range for the molecular weight of the high molecular weight substance is 1,000 to 2,000,000, preferably 5,000 or 50,000 or 170,000 to 100,000 or 220,000 or 500,000.

Examples of coagulating factor activating substance include the following:
(a) Reagent containing tissue thromboplastin.
   Prothrombin time reagent, reagent for measuring protein induced by vitamin K (reagent for measuring complex factor).
(b) Reagent containing phospholipid.
   Partial thromboplastin time reagent, activated partial thromboplastin time reagent.
(c) Reagent containing thrombin.
   Reagent for measuring fibrinogen, AT-III measuring reagent.

Thus, the coagulating factor activating substance may be a substance selected from the group comprising tissue thromboplastin, phospholipid and thrombin.

An example of reagent containing tissue thromboplastin is explained below. To prepare a reagent containing tissue thromboplastin, tissues (brain, lung, placenta, etc.) of mammal animals (human, rabbit, cattle, horse, monkey, etc.) are treated with acetone to obtain acetone treated powder (hereinafter called acetone powder), and fractions containing tissue thromboplastin are extracted from the acetone powder, and calcium ions and high molecular weight substances are added in the fractions.

An example of manufacturing process is as follows:
I. The brain of a rabbit is taken out.
II. Preparation of acetone powder.
   The rabbit brain is mixed and ground in acetone. The mixture is filtered and dried.
III. Removal of blood components and foreign proteins.
   The acetone powder is mixed in an acidic solution, and is centrifuged to obtain sediment fraction. At this time, the floating fraction contains blood components and foreign proteins.
IV. Extraction of tissue thromboplastin.
   The sediment fraction is mixed and stirred in solution, and centrifuged, and a floating fraction containing tissue thromboplastin is collected.
V. Addition of high molecular weight substances.
   Calcium ions and high molecular weight substances are added to the fraction containing tissue thromboplastin.
VI. Freeze-drying.
   The above reagent is freeze-dried.

At step II, meanwhile, it is desired to mix the rabbit brain and acetone at a ratio by weight of 1:7. The acetone powder is commercially available and may be bought.

Besides, at step III, by mixing acetone powder to an acidic solution (such as hydrochloric acid water), the undesired component may be much eluted in the solution, so that the removal rate of undesired component may be enhanced (blood corpuscles hemolyze in acidity).

At step IV, by using sodium formate or sodium acetate at a concentration of 25 to 250 mM, a tissue thromboplastin of high coagulating activity may be obtained efficiently.

The high molecular weight substances at step V may include, among others, high molecular weight vinyl selected from the group comprising polyethylene glycol, polyvinyl alcohol, and polynoxylene, or high molecular weight polysaccharide selected from the group comprising agarose, soluble starch, glycogen, dextran and dextrin. As the high molecular weight substance, high molecular weight vinyl, high molecular weight polysaccharide or both may be used. More specifically, for example, polyethylene glycol with molecular weight of 1,000 to 500,000 (more preferably 5,000 to 100,000, most preferably 20,000), or dextran with molecular weight of 5,000 to 2,000,000 (more preferably, 50,000 to 500,000, or most preferably 170,000 to 220,000) may be preferred. The content may be properly determined, but it is generally 0.25% to 2.0% (w/v), because the effect of high sensitivity is not obtained if too little, or the reaction performance deteriorates due to excessive viscosity if too much.

After adding the high molecular weight substance, by setting the reagent in the state at least satisfying one of the following conditions 1 to 3, the reagent of a higher sensitivity will be obtained (best when all three conditions are satisfied).
1. The electric conductivity is adjusted to 4.0 to 15 mS (more preferably 6.5 to 11.0 mS).
2. The pH is adjusted to 6.7 to 8.2 (more preferably 7.2 to 7.5).
3. The osmotic pressure is adjusted to 100 to 700 mmol/kg (100-700 mOsm/kg), more preferably 350 to 700 mmol/kg.

For adjustment of electric conductivity, an electrolyte may be used (such as salt). For adjustment of pH, acid or alkali is used (e.g. hydrochloric acid, sodium hydroxide). For adjustment of osmotic pressure, a substance which is non-electrolytic and water-soluble at the same time is used (e.g. sugar, urea). The prepared thromboplastin reagent should be preferably freeze-dried so as to be stored for a long period. Before use, the dry powder is dissolved in a specific amount of water to return to an initial state of solution.

It is thus preferred in the invention to use, as the coagulating factor activating substance, a fraction containing tissue thromboplastin taken out from the acetone treated powder obtained by treating mammal tissues with acetone. The fraction containing tissue thromboplastin is preferably obtained by dissolving the acetone treated powder in solution, mixing, stirring and centrifuging, or by floating the acetone treated powder in an acidic liquid, removing blood components and foreign proteins by centrifugation, adding liquid to the sediment fraction, stirring and then centrifuging.

The reagent containing tissue thromboplastin is described above, while the reagent containing phospholipid or the reagent containing thrombin may be obtained by employing the known art. As the reagent containing tissue thromboplastin, the prothrombin time reagent (PT reagent, the reagent for comprehensively measuring the extrinsic coagulation reaction in Fig. 1) is distributed by DADE, ORTHO, GD, all of the United States, Bering and BM, both of Germany, and others. As the reagent for measuring the activity of vitamin K derived enzyme (reagent for measuring complex factor), Thrombo Test and Hepaplastin Test (both tradenames) are distributed by Eisai of Japan, and Complex Factor H and Complex Factor T (both tradenames) are distributed by Kokusai Shiyaku of Japan.

As the reagent containing phospholipid, the APTT reagent and PTT reagent (the reagent for comprehensively measuring the intrinsic coagulation reaction in Fig. 1) are distributed by DADE, ORTHO, GD, all of the United States, Bering and BM, both of Germany. and others.

As the reagent containing thrombin, the reagent for measuring fibrinogen, reagent for measuring thrombin time, or reagent for measuring AT-III are distributed by DADE, ORTHO, GD, all of the United States, Bering and BM, both of Germany, Kokusai Shiyaku of Japan, and others.

Therefore, in the invention, as the coagulating factor activating substance, the phospholipid containing fraction obtained from acetone treated powder prepared by treating mammal tissue with acetone, or the thrombin containing fraction refined from plasma of mammal, may be used.

By mixing the blood coagulation reagent of the invention in the plasma, coagulation factors in the plasma are activated one after another, and finally fibrinogen is transformed into fibrin to terminate the coagulation reaction. In the final process, first monomers of fibrin are formed, and the monomers are gradually polymerized to form polymer (fibrous), so that the fibrin become fibrous. As the fibrin fibers are entangled like a mesh, the mixed solution becomes turbid and is gelled.

By tracing the optical characteristics (for example, intensity of scattered light) of the mixed solution sequentially from the point of mixing the reagent, the time until end of coagulation is known, from which the coagulation ability is calculated.

The reagent used in the invention contains high molecular weight substance. By the coexistence of the fiber and high molecular weight substance, the entangling is more complicated, and the difference in optical characteristic between right after mixing the reagent (before coagulation) and after coagulation reaction is greater than before (that is, higher in sensitivity).

Besides by varying the electric conductivity, pH or osmotic pressure, the quantity of optical change varies. By setting these values at proper values, a higher sensitivity is expected.

Thus, when the reagent used in the invention is mixed with the specimen plasma, the coagulation factors in the plasma are sequentially activated, and finally the fibrinogen is transformed into fibrin. At this time, for example, by detecting the turbidity change (increase of turbidity) optically, the coagulation time is measured, from which the coagulation ability is assessed (calculated).

In the final process of blood coagulation, the fibrinogen is transformed into fibrin, but initially monomers of fibrin are formed. As the reaction is advanced, the fibrin monomers grown into dimer and further into polymer (fiber) by polymerization reaction. In this process, the greater the quantity of fibers (fibrin) and the more complicated is the fiber entanglement, the greater is the turbidity change, and therefore the measurement of coagulation ability is enhanced in both accuracy and reproducibility. The blood coagulation reagent of the invention contains the high molecular weight substance in order to cause a greater change in the turbidity, and therefore it increases the hardness of coagulation owing to entangling of fibers and high molecular weight substance, as well as entangling of fibers, and amplifies the optical change quantity. Besides, by properly controlling the conditions such as electrical conductivity, the polymerization reaction from fibrin monomers to fibrin polymer may be encouraged. It is anyway effective to increase the turbidity change due to blood coagulation, so that a reagent of high sensitivity may be prepared.

The invention will now be described by way of non-limiting embodiments with reference to the accompanying drawings, in which:-
Fig. 1 is a block diagram showing the general pathways of blood coagulation;
Fig. 2 is a graph showing the change of signal during blood coagulation when using the conventional scattered light detection method;
Fig. 3 is a graph showing the relationship between the quantity of scattered light and time when using the coagulation reagent of the invention and when using a conventional coagulation reagent not containing a high molecular weight substance;
Fig. 4 is a graph showing the relationship between the electric conductivity and the change in the quantity of scattered light;
Fig. 5 is a graph showing the relationship between pH and the change in the quantity of scattered light; and
Fig. 6 is a graph showing the relationship between the osmotic pressure and the change in the quantity of scattered light.

For the reagent used in the method of examination called Quick's single-stage method (prothrombin time method), it is indispensable to contain tissue thromboplastin in order to endow the extrinsic coagulation factors with reactivity, and moreover in order that the result of reaction be measured accurately, it is necessary to set the reaction environments so that the coagulation lumps may be firmer. In an embodiment, accordingly, the tissue thromboplastin of high reactivity was extracted from the rabbit brain tissues in the following procedure.
(1) The rabbit was anesthetized with chloroform or other anesthetic, and the whole blood was extracted from the cervical artery. From the brain artery, normal (physiological) saline was perfused to rinse the blood in the brain. Then the cranium was opened, and the brain was removed (picked out).
(2) To 100g of rabbit brain obtained in this way, 700 ml of cold acetone was mixed, and the brain was ground and stirred by a mixer, and dehydrated and pulverized. The ground matter was filtered and dried in vacuum, and the acetone powder (AP) was obtained.
(3) 10g of AP was mixed in 100 ml of hydrochloric acid water (0.02 normal), and was centrifuged (3,000 rpm × 30 min) to collect sediment fractions. The separated and removed floating fractions contained blood components and foreign proteins.
(4) The collected sediment fractions were mixed and stirred in 100 ml of sodium formate (0.05 normal). By centrifugation (3,000 rpm × 30 min), floating fractions containing tissue thromboplastin were collected.
(5) To the collected floating fractions containing about 100 ml of tissue thromboplastin, 50 mM buffer agent for stabilizing the pH (phosphoric acid buffer solution, barbital buffer solution or tris buffer solution may be also usable), preservatives (0.1% w/v sodium azide), and stabilizer (0.1% calcium chloride) were mixed, and in order to make the coagulation reaction more manifest, in addition, a reagent was mixed, such as 0.5% polyethylene glycol (molecular weight 20,000) or 0.1% dextran (molecular weight 200,000). Furthermore, using sodium hydroxide of 0.1 normal, the pH was adjusted to 7.20 to 7.50, and the salt was mixed to adjust the electric conductivity to 6.5 to 10.0 mS, and glucose was added to adjust the osmotic pressure to 350 to 700 mmol/kg.
(6) Thus obtained reagent containing tissue thromboplastin was freeze-dried and stored.

The effect of removing blood components and foreign proteins by treating the acetone powder is explained. Before extracting the tissue thromboplastin, the acetone powder was dissolved in solution, and in order to investigate the effect of removing the blood components and foreign proteins, the deproteinized specimen (operation to wash acetone powder in acidic solution) and the control specimen (not deproteinized) were prepared, and using these PT reagents, ten measurements each were compared. As a result, as shown in Table 3, the deproteinized PT reagent was higher in coagulation activity than the control PT reagent without this treatment (that is, the coagulation time was shorter), and was superior in reproducibility.

It is hence known that the operation to wash the acetone powder in acidic solution prior to extraction of tissue thromboplastin is effective for enhancing the coagulation activity of PT reagent and for obtaining results of higher precision.

**Table 1**

| | Coagulation time | Reproducibility (variation factor) |
|---|---|---|
| Deproteinized | 14.58 sec | 0.52% |
| Not deproteinized | 16.52 sec | 1.86% |

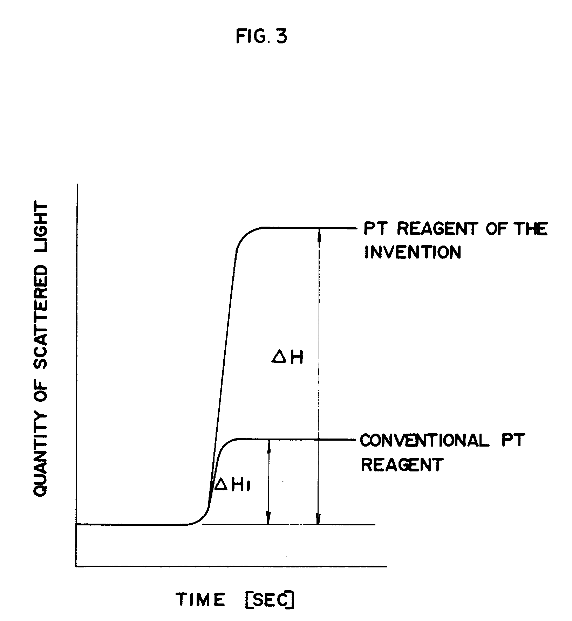

Still more, in order to see the effect of mixing high molecular substance in the reagent, the PT reagent containing tissue thromboplastin blended with high molecular substance and the PT reagent containing tissue thromboplastin without blend were prepared, and their coagulation curves were compared. As a result, as shown in Fig. 3, the PT reagent with high molecular substance was greater in the quantity of change of scattered light as compared with the PT reagent without the blend
(ΔH > ΔH₁).

It is hence known that the coagulation reaction can be analyzed more closely and precisely by mixing high molecular substance to the PT reagent. As the high molecular substance, ethylene glycol with molecular weight of 20,000 was used, and the blending rate was 0.5 w/v%. Meanwhile, ΔH denotes the change of scattered light by adding high molecular substance, and ΔH₁ represents the change of scattered light without high molecular substance.

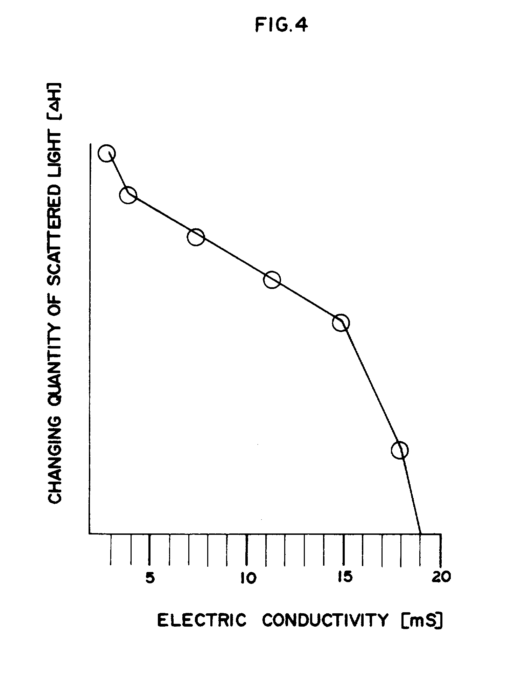

Fig. 4 shows the effect of adjusting the electric conductivity to 4.0 to 15.0 mS. To investigate the effect of adjusting the electric conductivity of the PT reagent to 4.0 to 15.0 mS, salt was added to tissue thromboplastin, and the PT reagent were prepared so that the electric conductivity be 2.0 to 20.0 mS, and using these PT reagents the plasma coagulation was measured, and the mean of changing quantity of scattered light (the mean of ten measurements each) was compared. As a result, as shown in Fig. 4, although the changing quantity of scattered light tended to decline as the electric conductivity elevated, there was a stabilizing tendency between 4.0 and 15.0 mS.

Hence, by adjusting the electric conductivity of the PT reagent to 4.0 to 15.0 mS, it is known that a stable change of scattered light may be obtained even if the electric conductivity varies depending on the plasma to be measured, while maintaining a high level of change of scattered light.

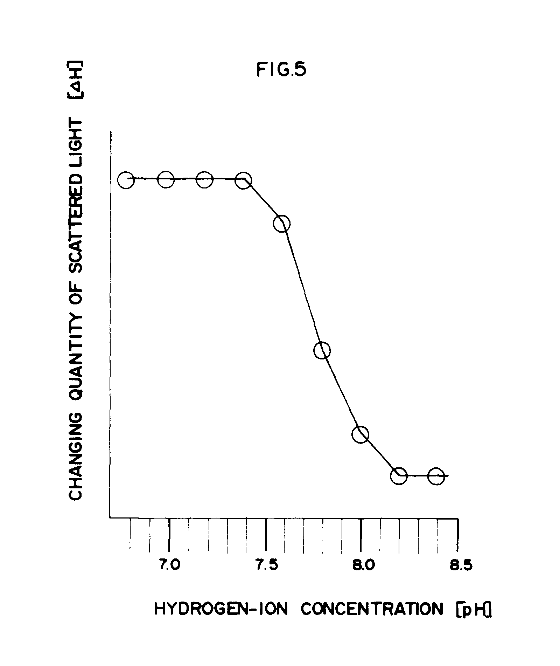

Fig. 5 shows the effect of adjusting the pH to 6.7 to 8.2, or preferably 7.2 to 7.5. To investigate the effect of adjusting the pH of the PT reagent to 6.7 to 8.2, or preferably 7.2 to 7.5, PT reagents were prepared in a pH range of 6.7 to 8.5 by using hydrochloric acid of 1 normal and sodium hydroxide of 1 normal in tissue thromboplastin extract, and the plasma coagulation was measured by using these PT reagents, and the mean of changing quantity of scattered light (the mean of ten measurements each) was compared. As a result, as shown in Fig. 5, at the pH of 6.7 to 7.5, the changing quantity of the scattered light tended to be stable at a relatively high level, but between pH 7.5 and 8.2, as the pH elevated, the changing quantity of scattered light tended to decline, and at pH 8.2 or higher, the changing quantity of scattered light tended to be stable at a relatively low level.

Hence, by adjusting the pH of the PT reagent within 6.7 to 8.2, or preferably 7.2 to 7.5, it is known that a stable changing quantity of scattered light is obtained regardless of fluctuations of pH depending on plasma specimens, while maintaining the changing quantity of scattered light at high level.

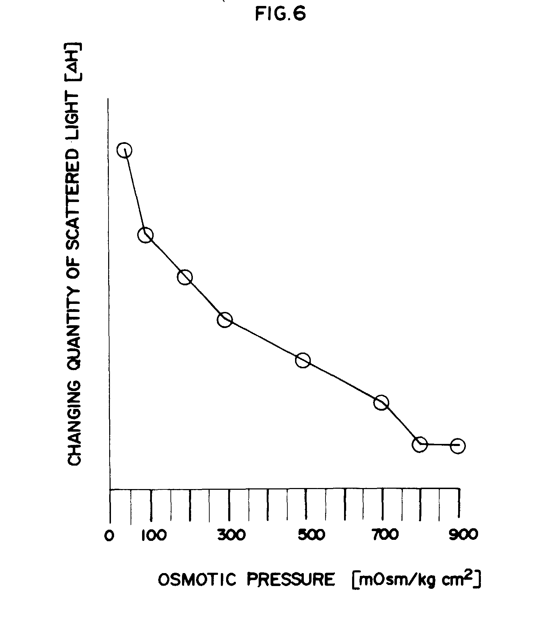

Fig. 6 shows the effect of adjusting the osmotic pressure to 100 to 700 mmol/kg, or preferably 350 to 700 mmol/kg. To see the effect of adjusting the osmotic pressure of PT reagent to 100 to 700 mmol/kg or preferably 350 to 700 mmol/kg, glucose was mixed to tissue thromboplastin extract, and reagents were prepared by adjusting the osmotic pressure in a range of 50 to 900 mmol/kg, and using these PT reagents, the plasma coagulation was measured, and the mean of the changing quantity of scattered light at that time (the mean of ten measurements each) was compared. As a result, as shown in Fig. 6, the changing quantity of scattered light tended to decline along with the elevation of osmotic pressure, but at the osmotic pressure of 100 to 700 mmol/kg, or in particular in a range of 350 to 700 mmol/kg, the changing quantity of scattered light tended to be stable.

Hence, by adjusting the osmotic pressure of the PT reagent to 100 to 700 mmol/kg, or preferably 350 to 700 mmol/kg, it is known possible to obtain a stable changing quantity of scattered light regardless of fluctuations of the osmotic pressure depending on specimen plasma, while maintaining the changing quantity of scattered light at a high level.

The reagent used in the invention to coagulate blood possesses reactivity to coagulation factors, same as the conventional coagulation factor activating reagent, and contains high molecular substance, and therefore it is effective to increase the turbidity change along with blood coagulation. By properly setting the conditions such as electric conductivity, the polymerization reaction from fibrin monomers to fibrin polymer can be encouraged, and it is also effective to increase the turbidity change along with blood coagulation.

These facts suggest that the formation state of fibrin lumps as the final product of blood coagulation is firm, and therefore in the measuring apparatus on the basis of the principle of detection of viscosity, the detectable range is extended even if the fibrinogen quantity is small, while the demerit of being influenced by intensity of magnetic substance or the like may be solved. In the turbidity detection method, the changing quantity of transmitted light or scattered light increases, so that a more accurate detection may be realized.

The description herein relates to the field of blood coagulation, but the method and reagent for adjusting the coagulation reaction environments and making the reaction manifest are not limited to the reagent used in the coagulation factor activating substance disclosed in the invention, but may be applied to everything that reacts to solidify or coagulate physically.

## Claims

1. Use of a high molecular weight substance of molecular weight between 1,000 and 2,000,000 in a reagent for coagulating blood comprising a substance activating coagulation factor and calcium ions, for increasing turbidity changes when said reagent is added to a plasma sample.

2. Use according to claim 1, wherein the high molecular weight substance is selected from polyethylene glycol, polyvinyl alcohol, polynoxyline, agarose, soluble starch, glycogen, dextran and dextrin.

3. Use according to either of claims 1 or 2, wherein the substance activating coagulation factor is a substance selected from tissue thromboplastin, phospholipid and thrombin.

4. Use according to any one of claims 1 to 3, wherein the substance activating coagulation factor is a fraction containing tissue thromboplastin obtained from acetone treated powder obtained by treating mammal tissues with acetone.

5. Use according to any one of claims 1 to 3, wherein the substance activating coagulation factor is a fraction containing phospholipid obtained from acetone treated powder obtained by treating mammal tissues with acetone.

6. Use according to any one of claims 1 to 3, wherein the substance activating coagulation factor is a fraction containing thrombin refined from mammal plasma.

7. Use according to claim 4, wherein the fraction containing tissue thromboplastin is obtained by dissolving acetone treated powder in solution, mixing, stirring and centrifuging.

8. Use according to claim 4, wherein the fraction containing tissue thromboplastin is obtained by floating acetone treated powder in acidic solution, centrifuging to remove blood components and foreign proteins, adding solutions to the sediment fraction, stirring and centrifuging.

9. Use according to any of claims 4, 7 and 8, wherein the electric conductivity of the reagent is adjusted to 4.0 to 15.0 mS.

10. Use according to any of claims 4, 7 and 8, wherein the final pH of the reagent is adjusted to 6.7 to 8.2.

11. Use according to any of claims 4, 7 and 8, wherein the osmotic pressure of the reagent is adjusted to 100-700 mmol/kg (100 to 700 mOsm/kg).

12. Use according to any preceding claim wherein the reagent contains 0.25 to 2.0 (w/v) % of high molecular weight substance.

## Patentansprüche

1. Verwendung einer hochmolekularen Substanz mit einem Molekulargewicht zwischen 1000 und 2 000 000 in einem Reagenz zur Blutgerinnung, welches eine den Gerinnungsfaktor aktivierende Substanz und Calciumionen umfaßt, zur Verstärkung der Trübungsveränderungen, wenn dieses Reagenz einer Plasmaprobe zugesetzt wird.

2. Verwendung nach Anspruch 1, bei der die hochmolekulare Substanz unter Polyethylenglycol, Polyvinylalkohol, Polynoxylin, Agarose, löslicher Stärke, Glycogen, Dextran und Dextrin ausgewählt ist.

3. Verwendung nach einem der Ansprüche 1 oder 2, bei der die den Gerinnungsfaktor aktivierende Substanz eine unter Gewebethromboplastin, Phospholipid und Thrombin ausgewählte Substanz ist.

4. Verwendung nach einem der Ansprüche 1 bis 3, bei der die den Gerinnungsfaktor aktivierende Substanz eine Gewebethromboplastin enthaltende Fraktion ist, die aus mit Aceton behandeltem Pulver erhalten wurde, welches durch Behandlung von Säugetiergeweben mit Aceton erhalten wurde.

5. Verwendung nach einem der Ansprüche 1 bis 3, bei der die den Gerinnungsfaktor aktivierende Substanz eine Phospholipid enthaltende Fraktion ist, die aus mit Aceton behandeltem Pulver erhalten wurde, welches durch Behandlung von Säugetiergeweben mit Aceton erhalten wurde.

6. Verwendung nach einem der Ansprüche 1 bis 3, bei der die den Gerinnungsfaktor aktivierende Substanz eine aus Säugetierplasma gereinigtes Thrombin enthaltende Fraktion ist.

7. Verwendung nach Anspruch 4, bei der die Gewebethromboplastin enthaltende Fraktion durch Auflösen von mit Aceton behandeltem Pulver in Lösung, Vermischen, Rühren und Zentrifugieren erhalten ist.

8. Verwendung nach Anspruch 4, bei der die Gewebethromboplastin enthaltende Fraktion erhalten wurde, indem man mit Aceton behandeltes Pulver in saurer Lösung zum Schwimmen bringt, zentrifugiert, um Blutbestandteile und Fremdproteine zu entfernen, Lösungen zu der Sedimentfraktion zusetzt, rührt und zentrifugiert.

9. Verwendung nach einem der Ansprüche 4, 7 und 8, bei der die elektrische Leitfähigkeit des Reagenz auf 4,0 bs 15,0 mS eingestellt wird.

10. Verwendung nach einem der Ansprüche 4, 7 und 8, bei der der End-pH-Wert des Reagenz auf 6,7 bis 8,2 eingestellt wird.

11. Verwendung nach einem der Ansprüche 4, 7 und 8, bei der der osmotische Druck des Reagenz auf 100 bis 700 mmol/kg (100 bis 700 mOsm/kg) eingestellt wird.

12. Verwendung nach einem der vorausgehenen Ansprüche, bei der das Reagenz 0,25 bis 2,0 % (Gew./Vol.) der hochmolekularen Substanz enthält.

## Revendications

1. Utilisation d'une substance de poids moléculaire élevé, d'un poids moléculaire compris entre 1 000 et 2 000 000, dans un réactif pour coaguler le sang, comprenant une substance activant le facteur de coagulation et des ions calcium, pour augmenter des changements de turbidité quand ledit réactif est ajouté à un échantillon de plasma.

2. Utilisation selon la revendication 1, dans laquelle la substance de poids moléculaire élevé est choisie parmi les polyéthylèneglycol, alcool polyvinylique, polynoxyline, agarose, amidon soluble, glycogène, dextrane et dextrine.

3. Utilisation selon la revendication 1 ou 2, dans laquelle la substance activant le facteur de coagulation est une substance choisie parmi la thromboplastine tissulaire, un phospholipide et la thrombine.

4. Utilisation selon l'une quelconque des revendications 1 à 3, dans laquelle la substance activant le facteur de coagulation est une fraction contenant de la thromboplastine tissulaire obtenue à partir d'une poudre traitée à l'acétone, obtenue par traitement de tissus de mammifères avec de l'acétone.

5. Utilisation selon l'une quelconque des revendications 1 à 3, dans laquelle la substance activant le facteur de coagulation est une fraction contenant un phospholipide obtenu à partir d'une poudre traitée à l'acétone, obtenue par traitement de tissus de mammifères avec de l'acétone.

6. Utilisation selon l'une quelconque des revendications 1 à 3, dans laquelle la substance activant le facteur de coagulation est une fraction contenant de la thrombine purifiée à partir de plasma de mammifère.

7. Utilisation selon la revendication 4, dans laquelle la fraction contenant de la thromboplastine tissulaire est obtenue par dissolution d'une poudre en solution traitée à l'acétone, mélange, agitation et centrifugation.

8. Utilisation selon la revendication 4, dans laquelle la fraction contenant de la thromboplastine tissulaire est obtenue par flottation d'une poudre traitée à l'acétone dans une solution acide, par centrifugation pour séparer les composants sanguins et des protéines étrangères, par addition de solutions à la fraction de sédiment, agitation et centrifugation.

9. Utilisation selon l'une quelconque des revendications 4, 7 et 8, dans laquelle la conductivité électrique du réactif est ajustée entre 4,0 et 15,0 mS.

10. Utilisation selon l'une quelconque des revendications 4, 7 et 8, dans laquelle le pH final du réactif est ajusté entre 6,7 et 8,2.

11. Utilisation selon l'une quelconque des revendications 4, 7 et 8, dans laquelle la pression osmotique du réactif est ajustée entre 100 et 700 mmol/kg (entre 100 et 700 mOsm/kg).

12. Utilisation selon l'une quelconque des revendications précédentes, dans laquelle le réactif contient de 0,25 à 2,0 % (p/v) de substance de poids moléculaire élevé.
